# EUROPEAN PATENT SPECIFICATION

(11) **EP 1 570 853 B1**
(45) Date of publication and mention of the grant of the patent: **22.08.2007**
(21) Application number: 05101687.1
(22) Date of filing: 04.03.2005
(51) Int. Cl.: A61K 35/54

(54) **Product with anti-psoriatic activity**
Produkt mit antipsoriatischer Aktivität
Produit avec l'activité anti-psoriasique

(30) Priority: 04.03.2004 IT MI20040423
(43) Date of publication of application: 07.09.2005
(73) Proprietor: Biava, Piermario, 20123 Milano (IT); Ricotti, Annamaria, 20123 Milano (IT)
(72) Inventor: Biava, Piermario, 20123 Milano (IT); Ricotti, Annamaria, 20123 Milano (IT)
(74) Representative: Gervasi, Gemma

(56) References cited:
- EP-A- 1 159 966
- GB-A- 1 388 324
- DATABASE WPI Section Ch, Week 199947 Derwent Publications Ltd., London, GB; Class B04, AN 1999-551718 XP002333548 & CN 1 223 117 A (LU G) 21 July 1999 (1999-07-21)

## Description

### PRIOR ART

Extracts from embryos or gravid uteri are known from the prior art and their use as anti-tumour substances has been described in a number of patent applications.

For example, French patent A-2451193, describes the use of a homogenate composed of a hydroalcoholic extract from cow, sheep or swine embryos for treatment of tumours.

Patent application WO 98/11905 discloses a composition suitable for tumour treatment containing an extract of embryos or gravid uterus, wherein the embryos are isolated from oviparous or ovoviviparous animals and the gravid uterus is isolated from mouse, rabbit, sheep or pig.

EP0929308 discloses that embryo extracts removed at specific stages of cellular differentiation are effective for the treatment of malignancies and of other pathologies controlled by the anti-oncogene p53, and that said specific stages of embryonic development change are dependent upon the species of origin of the extracts.

Furthermore, EP1374703 describes that extracts from non-human animal embryos, removed at specific and appropriate stages of differentiation during development, are effective as nutritive substances, exerting systemic effects, and as topical products for nutrition of the skin and cutaneous appendages.

WPI Section Ch, Week 199947 Derwent Publications Ltd., London, GB; Class B04, AN 1999-551718 XP002333548 & CN 1 223 117 A (LU G) 21 July 1999 (1999-07-21) teaches mixtures of an embryonic extract from oviparous birds and of an extract from human placenta.

EP1159966 discloses an alcoholic extract of human placenta and the uses thereof for treating psoriasis.

### SUMMARY OF THE INVENTION

We have now found that extracts from non-human mammalian decidua or placenta are unexpectedly able to selectively block the activity of activated T-lymphocytes, while it does not have any effect on normal T-lymphocytes.

We have also surprisingly found that, thanks to this mechanism of action, when the above extracts are combined with specific embryonic extracts they unexpectedly result clinically very effective in the therapy or support treatment of psoriasis.

Thus, the present invention relates to a mixture of an extract obtained from oviparous embryos and an extract obtained from non-human mammalian decidua or placenta. The present invention also relates to the use of the above mixture in the preparation of a composition for the treatment of psoriasis and to cosmeceutical, nutraceutical or medical device compositions for topical use containing the above mixture.

### DETAILED DESCRIPTION OF THE INVENTION

The present invention refers to a mixture of:
a) an extract from a non-human mammalian decidua or placenta, isolated during the cellular differentiation period, with
b) an extract from a mixture of oviparous embryos consisting of 66 wt% of embryos at the midblastula-gastrula stage, 17 wt% of embryos at the five-somite stage and 17 wt% of embryos at the twenty-somite stage.

Embryos suitable for the uses according to the present invention are isolated from fishes, birds and amphibians.

According to preferred embodiments of the present invention, when the embryos are isolated from fishes, these are preferably selected from the group consisting of brachydanio rerio, trout, carp and salmon; when the embryos are isolated from birds, these are preferably selected from chicken and quails; finally, when the embryos are isolated from amphibians, these are preferably isolated from frogs.

Preferably, the decidua or placenta are isolated from a non-human mammalian selected from the group consisting of mouse, pig, rabbit, sheep, goat, cow and horse.

The mixture of the invention is prepared by a method comprising the following steps:
I) preparation of the extract b) from a mixture of oviparous embryos;
II) preparation of the extract a) from decidua or placenta; and
III) mechanical mixture of the extracts obtained in step I) and step II)

A method suitable for obtaining the embryonic extract b) is described herein below.

A mixture of embryos is prepared having the following composition: 66 wt% of embryos at the midblastula-gastrula stage, 17 wt% of embryos at the five-somite stage and 17 wt% of embryos at the twenty-somite stage, they are washed in distilled water and then suspended in a solution consisting of 85% glycerol and 15% ethyl alcohol 98%.

The suspension is then treated with 1 to 4, preferably two, cycles of ultrasounds of 10-20 seconds each, preferably of 10 seconds, and then treated in a turboemulsifier for 1 to 5 minutes, preferably 3 minutes. Such suspension is diluted with purified water or with a mixture of 70% purified water and 30% of ethyl alcohol in a volume ratio of 1:10 and is then filtered under vacuum through a 90 micrometer Millipore membrane and subsequently through a 10 micrometer membrane. A solution is obtained having a total concentration of embryonic proteins ranging from 3 to 100 micrograms/ml.

The solution thus obtained may be optionally freeze-dried and stored until use.

For the preparation of extract a) from non-human mammalian decidua or placenta, it is necessary to take into account the different animal species used, considering that such extracts must be prepared during the most significant period of cellular differentiation.

For instance, for the mouse, said period lies between the 4th and 11th day of gestation. For the pig, said period lies between the 7th and the 40th day of gestation. For the horse, said period lies between the 10th and the 55th day of gestation, even though complete differentiation of some cutaneous appendages occurs after such period.

The extract a) may be either a complete extract from decidua or placenta, comprising all molecular weight fractions of it or only a partial extract consisting of the fraction of the extract from decidua or placenta having molecular weight lower than 10,000 Daltons.

Preferably, extract a) is obtained with the process described herein below.

In the case of extracts from decidua, the gravid uterus is isolated from a non-human mammalian during the described period, the embryo is removed from the gravid uterus and the uterus portion in which the embryo was implanted isolated.

The decidua or placenta extract is then be prepared by the following procedure, which is essentially identical starting from decidua or placenta.

The placenta or decidua isolated from a non-human mammalian is treated with a physiological solution, preferably, PBS (phosphate saline buffer), in a homogenizer.

The ratio between physiological solution and decidua or placenta is preferably comprised between 5 and 20 ml of PBS for 100mg of decidua or placenta, and the homogenization procedure is preferably repeated for 2 to 5 times, preferably for 3 times, each time for the duration of 1 to 2 minutes, preferably of 1 minute.

The homogenized mixture is then centrifuged at 1500 to 3000, preferably 2000, revolutions per minute for 3-6 minutes, preferably 5 minutes.

The supernatant layer is taken and filtered.

The filtrate is diluted with purified water in a volume ratio from 1:1 to 1:8.

In case it is desired to obtain a complete extract of decidua or placenta the solution thus obtained is used as such.

In case, alternatively, it is desired to a obtain only the fraction having molecular weight lower that 10,000 Daltons the solution may be subjected to a further step of fractionation with molecular sieves separating the molecular fraction with molecular weight lower than 10,000 Daltons.

The total crude extract or the 10,000 Daltons fraction from decidua or placenta, obtained by the above methods, are re-suspended in a solution composed of 85% pure glycerol and 15% ethyl alcohol.

The suspension is subsequently subjected to 1 to 4, preferably 2, cycles of sonication, of 10 to 20 seconds each, preferably of 10 seconds, and is then treated in a turboemulsifier, for 1 to 5 minutes, preferably for 3 minutes.

The resulting suspension is diluted with purified water or with a mixture of 70% purified water and 30% of ethyl alcohol in a volume ratio of 1:10 and is then filtered under vacuum through a 90 micrometer Millipore membrane and subsequently through a 10 micrometer membrane.

Also in this case the solution obtained may be freeze-dried and stored until use.

According to a preferred embodiment, in the mixture of the invention the decidua or placenta extract a) and the embryonic extract b) are in a weight ratio of 1:1.

As will be demonstrated in the experimental section, the present inventors have found that topical applications of compositions containing the mixture of the invention are clinically very effective in the therapy or support treatment of psoriasis.

Therefore, another object of the present invention is the use of the mixture of the invention in the preparation of a composition for topical use for the therapy or support treatment of psoriasis.

Furthermore, the present invention also refers to compositions for topical use, useful in the treatment or support treatment of psoriasis, containing as an active ingredient a therapeutically effective amount of the mixture of the invention in combination with biologically acceptable diluents and/or excipients. The compositions of the invention may be in any form suitable for topical application, for example cream, ointment, lotion foam or gel.

Preferably, the compositions of the invention contain an amount of the mixture of the invention comprised between 10% and 25% and more preferably of 14%.

The compositions of the invention may be cosmeceutical, nutraceutical or medical device compositions.

Some preparations of compositions according to the present invention are reported hereinafter. In this context, EPEP refers to extracts from brachydanio rerio embryos, obtained by the procedure described in the present application, and

MUG refers to extracts from decidua obtained from pig at the 24th day of pregnancy, according to the above described procedure.

The compositions given below are only some examples of possible embodiments of the present invention, whereas other compositions are possible by changing the percentage of EPEP and MUG and using excipients and diluents in different ratios depending on the type of psoriasis.

### Example 1

### a)Preparation of an extract from brachydanio rerio embryos (EPEP extract)

A mixture of embryos from brachydanio rerio embryos was obtained having the following composition:
66 wt% of embryos at the midblastula-gastrula stage,
17 wt% of embryos at the five somite stage, and
17 wt% of embryos at the twenty somite stage.

The mixture was washed with distilled water and suspended in a solution consisting of 85% glycerol and 15 of ethyl alcohol 98%.

The suspension obtained was treated with 2 cycles of ultrasounds of 10 seconds each and then treated in a turboemulsifier for 3 minutes. The suspension was diluted 10 times with a mixture of 70% water and 30% ethyl alcohol and then filtered under vacuum through a 90 micrometer Millipore membrane and subsequently through a 10 micrometer membrane.

### b) Preparation of an extract from decidua of pig (MUG extract)

The decidua was obtained from a pig at the 24th day of pregnancy.

100 mg of decidua were suspended in 10 ml of PBS and homogenised 3 times for 1 minute. The homogenised mixture was then centrifuged at 2000 revolutions for 5 minutes. The supernatant was collected, filtered and diluted 1:4 with purified water. The diluted filtrate was suspended in a solution consisting of 85% glycerol and 15% ethyl alcohol, subjected to two cycles of sonication of 10 seconds each and subsequently treated with a turboemulsifier for 3 minutes.

The resulting suspension was diluted 10 times in 70% purified water/ 30% ethyl alcohol, then filtered under vacuum through a 90 micrometer Millipore membrane and subsequently through a 10 micrometer membrane.

### Example 2

### Preparation of a composition in cream form

Xalofin™, Nesatol™, glycerin, Euxil™ were melted together in a controlled temperature container set at 70 °C. Water was heated separately to 80° C. The above mixture was added to the heated water and the obtained suspension cooled under conditions of vigorous agitation. When the suspension was equilibrated to 30 °C, EPEP and MUG obtained in Example 1 above were added and the suspension cooled to 25 °C

The amounts of the various substances used in the preparation were the following:

| | |
|---|---|
| EPEP | 7% |
| MUG | 7% |
| Xalofin 15™ (VEVY-Genoa) | 10% |
| Nesatol^{™} (VEVY-Genoa) | **9%** |
| Glycerin | 3% |
| Euxil K 400 (Schulke-Majr Milan) | 0.2% |
| Perfume | 0.1% |
| Purified water remainder to | 100% |

### Example 3

### Preparation of a composition in gel form.

EPEP and MUG, obtained in example 1 above, were diluted with distilled water, phosphatidylcholine was added and the mixture agitated using a turbine to produce a liposomal dispersion.

Glycerin was added under agitation to the dispersion and then the thickening agent Carbopol™ was added.

Agitation was continued until Carbopol™ is completely hydrated.

The amounts of the various substances used in the preparation were the following:

| | |
|---|---|
| EPEP | 7% |
| MUG | 7% |
| Ethyl Alcohol | 10% |
| Phosphatidylcholine | 0.5% |
| Glycerin | 3% |
| Carbopol 940™ (Goodrich-Milan) | 1% |
| Purified water remainder to | 100% |

### Example 4

### Preparation of composition in lotion form

Distilled water, ethyl alcohol, Glycerol, Panthenol, EPEP and MUG were placed in a container equipped with an agitator.

The solution thus obtained was filtered through a 0.45 micrometer membrane

The amounts by weight of the various substances used in the preparation were the following:

| | |
|---|---|
| EPEP | 7% |
| MUG | 7% |
| Ethyl Alcohol | 20% |
| Glycerol | 3% |
| Panthenol | 0,1% |
| Purified water remainder to | 100% |

### Example 5

Application trials of the compositions of the invention.

The effectiveness of the use of extracts from oviparous embryos and non-human mammalian gravid uterus of the present invention for therapeutic purpose or as support medical device or as nutraceutical product in the management of psoriasis is demonstrated by the following application trial:
Clinical trial on 30 subjects with psoriasis, including 10 subjects with spot or plaque psoriasis, 8 subjects with scalp psoriasis, 10 subjects with palmoplantar psoriasis and 2 subjects with guttate psoriasis.
All subjects were treated for 2 months with preparations as in the example 3.
After 15 days of treatment, 80% of the subjects showed a remarkable improvement of clinical symptoms. In particular, a reduction and, in few cases, the disappearance of scales and a reduction of the rash occurred in 8 subjects with plaque psoriasis and in 8 subjects with palmoplantar psoriasis. Two subjects with guttate psoriasis showed reduction of the rash until skin normalisation. Six subjects with scalp psoriasis had remarkable reduction of itching, reduction of the spots and of the erythema.
Such an improvement was generally achieved after two weeks of treatment and was maintained as such for two months, during which time the therapy was continued.

## Claims

1. A mixture of:
a) an extract from a non-human mammalian decidua or placenta, isolated during the cellular differentiation period, and
b) an extract from a mixture of oviparous embryos consisting of 66 wt% of embryos at the midblastula-gastrula stage, 17 wt% of embryos at the five-somite stage and 17 wt% of embryos at the twenty-somite stage.

2. A mixture as claimed in claim 1 wherein said embryos are isolated from a fish, bird or amphibian selected from the group consisting of brachydanio-rerio, trout, carp, salmon, chicken, quail and frog.

3. A mixture as claimed in claims 1 or 2, wherein the extract a) and the extract b) are in a weight ratio of 1:1.

4. A mixture as claimed in claims 1 to 3 wherein said decidua or placenta are isolated from a non-human mammalian selected from the group consisting of mouse, pig, rabbit, sheep, goat, cow and horse.

5. A mixture as claimed in claims 1 to 4 wherein said decidua or placenta is isolated from a non-human mammalian selected from the group consisting of the mouse between the 4th and 11th day of gestation, the pig between the 7th to the 40th day of gestation, the horse between the 10th to the 55th day of gestation and the cow between the 7th to the 40th day of gestation.

6. A mixture as claimed in claims 1 to 4 wherein said extract a) from decidua or placental is a complete extract from decidua or placenta.

7. A mixture as claimed in claims 1 to 4 wherein said extract a) from decidua or placental is a partial extract consisting of the fraction of the extract from the decidua or placenta having molecular weight lower than 10,000 Daltons.

8. Use of a mixture according to claims 1 to 7 for the preparation of a composition for topical use for the therapy of psoriasis or for the support treatment, as nutraceutical or cosmeceutical or medical device composition, of psoriasis.

9. A composition for topical use containing as an active ingredient a therapeutically effective amount of a mixture according to claims 1 to 7 in combination with biologically acceptable diluents and/or excipients.

10. A composition according to claim 9 in the form of a cream, ointment, lotion, foam or gel.

11. A composition according to claims 9 or 10 containing said mixture in an amount comprised between 10% and 25%.

12. A composition according to claim 11 containing said mixture in an amount of 14%.

13. A method for obtaining a mixture as claimed in claims 1 to 7 comprising the following steps:
I) preparation of the extract b) from said mixture of oviparous embryos;
II) preparation of the extract a) from said decidua or placenta; and
III)mixing of the extracts obtained in step I) and step II).

## Patentansprüche

1. Mischung von:
a.) einem Extrakt aus einer Dezidua oder Plazenta von einem nicht-humanen Säugetier, welche während der Phase der Zelldifferenzierung isoliert wird, und
b.) einem Extrakt aus einer Mischung von Embryonen aus Eiern, welcher aus 66 Gew.% an Embryonen im Midblastula-Gastrula Stadium, 17 Gew.% an Embryonen im Fünf-Somiten-Stadium and 17 Gew.% an Embryonen im Zwanzig-Somiten-Stadium besteht.

2. Mischung wie in Anspruch 1 beansprucht, wobei genannte Embryonen aus einem Fisch, Vogel oder Amphibium isoliert werden, ausgewählt aus der Gruppe, die aus Brachydanio rerio, Forelle, Karpfen, Lachs, Huhn, Wachtel und Frosch besteht.

3. Mischung wie in Anspruch 1 oder 2 beansprucht, wobei der Extrakt a) und der Extrakt b) ein Gewichtsverhältnis von 1:1 aufweisen.

4. Mischung wie in Ansprüchen 1 bis 3 beansprucht, wobei genannte Dezidua oder Plazenta aus einem nicht-humanen Säugetier isoliert wird, das aus der Gruppe ausgewählt ist, die aus Maus, Schwein, Kaninchen, Schaf, Ziege, Kuh und Pferd besteht.

5. Mischung wie in Ansprüchen 1 bis 4 beansprucht, wobei genannte Dezidua oder Plazenta aus einem nicht-humanen Säugetier isoliert wird, welches aus der Gruppe ausgewählt ist, die aus der Maus zwischen dem 4. und 11. Tag der Trächtigkeit, dem Schwein zwischen dem 7. und dem 40. Tag der Trächtigkeit, dem Pferd zwischen dem 10. und dem 55. Tag der Trächtigkeit und der Kuh zwischen dem 7. und dem 40. Tag der Trächtigkeit besteht.

6. Mischung wie in Ansprüchen 1 bis 4 beansprucht, wobei es sich bei dem genannten Extrakt a) aus Dezidua oder Plazenta um einen Gesamtextrakt aus Dezidua oder Plazenta handelt.

7. Mischung wie in Ansprüchen 1 bis 4 beansprucht, wobei es sich bei dem genannten Extrakt a) aus Dezidua oder Plazenta um einen Teilextrakt handelt, welcher aus dem Anteil des Extrakts aus der Dezidua oder der Plazenta mit einem Molekulargewicht von unter 10.000 Daltons besteht.

8. Verwendung einer Mischung gemäß Ansprüchen 1 bis 7 für die Herstellung einer Zusammensetzung für den topischen Gebrauch zur Therapie von Psoriasis oder zur unterstützenden Behandlung von Psoriasis als nutrazeutische oder kosmezeutische Zusammensetzung oder Zusammensetzung für eine medizinische Vorrichtung.

9. Zusammensetzung zur topischen Verwendung, welche als einen aktiven Inhaltsstoff eine therapeutisch wirksame Menge einer Mischung gemäß Ansprüchen 1 bis 7 in Kombination mit biologisch anerkannten Verdünnungsmitteln und/oder Trägerstoffen enthält.

10. Zusammensetzung gemäß Anspruch 9 in der Form einer Creme, Salbe, Lotion, eines Schaums oder Gels.

11. Zusammensetzung gemäß Anspruch 9 oder 10, welche genannte Mischung in einer Menge enthält, die zwischen 10 % und 25 % umfasst.

12. Zusammensetzung gemäß Anspruch 11, welche genannte Mischung in einer Menge von 14 % enthält.

13. Verfahren zum Erhalten einer Mischung, wie in Ansprüchen 1 bis 7 beansprucht, welches die folgenden Schritte umfasst:
I) Herstellung von dem Extrakt b) aus genannter Mischung von Embryonen aus Eiern;
II) Herstellung von dem Extrakt a) aus genannter Dezidua oder Plazenta, und
III) Mischung aus den Extrakten, welche in Schritt I) und Schritt II) erhalten werden.

## Revendications

1. Mélange de:
a) un extrait d'une caduque ou d'un placenta de mammifère non humains, isolés pendant la période de différenciation cellulaire, et
b) un extrait d'un mélange d'embryons d'ovipares consistant en 66 % en poids d'embryons au stade mi-blastula - gastrula, 17 % d'embryons au stade cinq somites et 17 % d'embryons au stade vingt somites.

2. Mélange selon la revendication 1 dans lequel lesdits embryons sont isolés sur des poissons, des oiseaux ou des amphibiens choisis dans le groupe consistant en *brachydanio rerio,* la truite, la carpe, le saumon, le poulet, la caille et la grenouille.

3. Mélange selon les revendications 1 ou 2, dans lequel l'extrait a) et l'extrait b) sont présents suivant un ratio en poids de 1:1.

4. Mélange selon les revendications 1 à 3 dans lequel ladite caduque ou ledit placenta sont isolés sur un mammifère non humain choisi dans le groupe consistant en une souris, une truie, une lapine, une brebis, une chèvre, une vache et une jument.

5. Mélange selon les revendications 1 à 4 dans lequel ladite caduque ou ledit placenta sont isolés sur un mammifère non humain choisi dans le groupe consistant en la souris entre le 4^{e} et le 11^{e} jour de gestation, la truie entre le 7^{e} et le 40^{e} jour de gestation, la jument entre le 10^{e} et le 55^{e} jour de gestation et la vache entre le 7^{e} et le 40^{e} jour de gestation.

6. Mélange selon les revendications 1 à 4 dans lequel ledit extrait a) de caduque ou de placenta est un extrait complet de caduque ou de placenta.

7. Mélange selon les revendications 1 à 4 dans lequel ledit extrait a) de caduque ou de placenta est un extrait partiel consistant en la fraction de l'extrait de caduque ou de placenta ayant un poids moléculaire inférieur à 10 000 daltons.

8. Utilisation d'un mélange selon les revendications 1 à 7 pour la préparation d'une composition à usage topique pour le traitement du psoriasis ou pour le traitement de soutien, comme produit neutraceutique ou cosméceutique ou comme composition de dispositif médical, du psoriasis.

9. Composition à usage topique contenant comme ingrédient actif une quantité efficace sur le plan thérapeutique d'un mélange selon les revendications 1 à 7 en combinaison avec des diluants et/ou des excipients biologiquement acceptables.

10. Composition selon la revendication 9 sous la forme d'une crème, d'un onguent, d'une lotion, d'une mousse ou d'un gel.

11. Composition selon les revendications 9 ou 10 contenant ledit mélange en une quantité comprise entre 10 % et 25 %.

12. Composition selon la revendication 11 contenant ledit mélange en une quantité de 14 %.

13. Procédé pour obtenir un mélange selon les revendications 1 à 7 comprenant les étapes suivantes:
I) préparation de l'extrait b) à partir dudit mélange d'embryons d'ovipare;
II) préparation de l'extrait a) à partir de ladite caduque ou dudit placenta; et
III) mélange des extraits obtenus à l'étape I) et à l'étape II).
